# EUROPEAN PATENT APPLICATION

(11) **EP 1 674 114 A1**
(43) Date of publication of application: **28.06.2006**
(21) Application number: 05028073.4
(22) Date of filing: 21.12.2005
(51) Int. Cl.: A61L 9/20, A61L 9/22, F24F 3/00

(54) **Air sterilizing unit for ventilation convectors and the like**

(30) Priority: 23.12.2004 IT MI20042498
(71) Applicant: KOVER S.r.l., 20122 Milano (IT)
(72) Inventor: Tomaselli, Giancarlo, 20122 Milano (IT)
(74) Representative: Cicogna, Franco

(57) **Abstract**

An air sterilizing unit, specifically designed for ventilation convectors and the like, comprises a first construction including sterilizing means which can be coupled to a second construction including control means for controlling the sterilizing means, the air sterilizing unit, comprising the mentioned two assembled constructions, being adapted to be engaged in a ventilation convector, in replacement of a conventional anti-powder filter.

## Description

### BACKGROUND OF THE INVENTION

The present invention relates to an air sterilizing unit, specifically designed for ventilating convectors and the like.

As is known, ventilating convectors consist of apparatus or devices used for air climatizing offices, buildings, hotels, schools and the like.

A conventional ventilating convector, or "fancoil", comprises a fancoil construction supporting and including therein a thermal exchange battery, an electrofan, an air filter and an adjusting and controlling system.

Said fancoils are made according to different embodiments, depending on their installation arrangement such as horizontal, vertical, recessed, with suction from the bottom, from the front and vertical/horizontal, and have a size which depends on the desired air flow rate.

Commercially available ventilating convectors or fancoils usually comprise and anti-powder or anti-dust filter, arranged at a suction grill which operates to prevent powder or dust from propagating and for protecting the thermal exchange batteries or banks.

Also known is the fact that fancoil filters are affected by a serious drawback of constituting a growth field for bacteria and other microorganisms, with a consequent diffusion of these agents through the environment, if their filters are not properly replaced or cleaned: in fact, if said filters are not properly replaced or cleaned, the mentioned microorganisms can diffuse through the outside or room environment, and cause lung diseases and the like.

### SUMMARY OF THE INVENTION

Accordingly, the aim of the present invention is to provide an air sterilizing unit, to be applied to fancoils for replacing the conventional anti-powder or anti-dust filter, adapted to assure an efficient microbiologic controlled reduction.

Within the scope of the above mentioned aim, a main object of the present invention is to provide such a modular air sterilizing unit, which can be applied to different types and size existing fancoils.

Another object of the invention is to provide such an air sterilizing unit the operation of which can be easily controlled, and which unit, moreover, can be quickly and easily serviced.

According to one aspect of the present invention, the above mentioned aim and objects, as well as yet other objects, which will become more apparent hereinafter, are achieved by an air sterilizing unit, particularly for fancoils and the like, characterized in that said unit comprises a second construction including air sterilizing means, said second construction being associated with a first construction including control means for controlling said air sterilizing means.

The sterilizing unit, comprising the two above mentioned assembled constructions, can be arranged in a fancoil, in replacement of a conventional anti-powder filter.

In this connection it should be pointed out that the air sterilizing means comprise anti-germ lamps, and an air ionizing emitter.

The control means comprise: an electronic hour or time counter 6, an electronic power supply 7, designed for power supplying lamps, an ionizer power supply 8, and a high frequency source 17 (or air ionizer).

Moreover, the control means comprise an electronic device for controlling the lamp switching on and off, said electronic device including a small casing arranged on the first construction.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:
Figure 1 is an exploded perspective view of a fancoil including an air sterilizing unit according to the present invention;
Figure 2 is a further perspective view of the fancoil shown in figure 1, including the air sterilizing unit according to the invention, in an operating condition thereof;

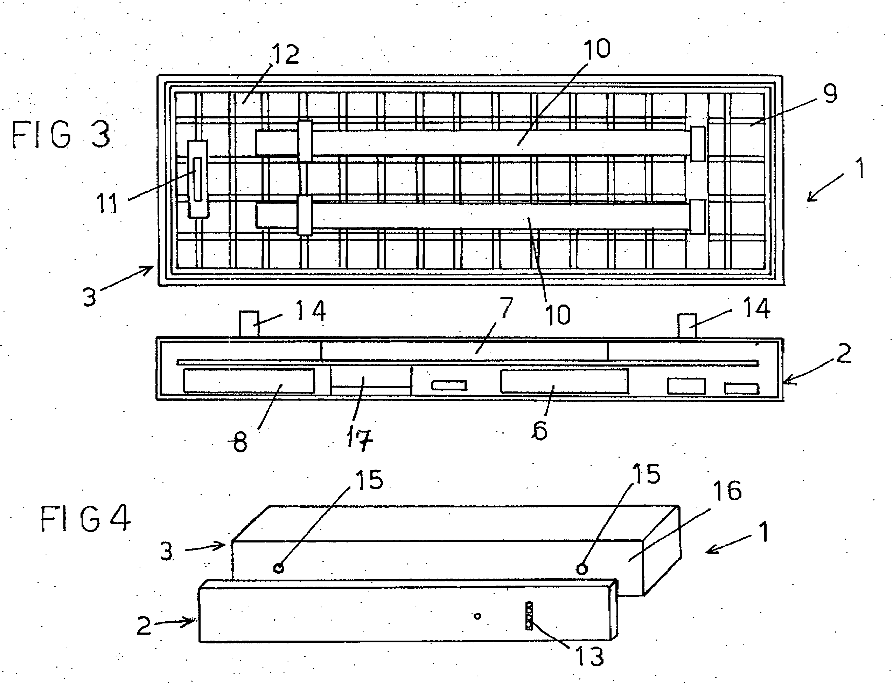

Figure 3 is a further top plan exploded view of the air sterilizing unit; and
Figure 4 is yet another exploded perspective view of said air sterilizing unit.

### DESCRIPTION OF THE PREFERRED EMBODIMENT

With reference to the number references of the above mentioned figures, the air sterilizing unit, generally indicated by the reference number 1, comprises a first construction 2 including specifically designed control means.

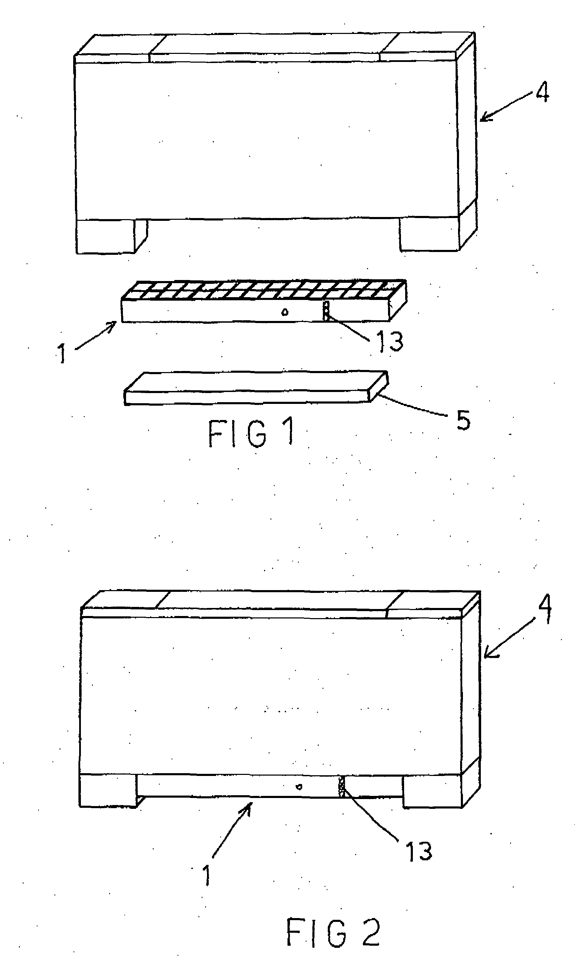

Said first construction 2 can be associated with or coupled to a second construction 3 including the air sterilizing means, and can be applied to a ventilation convector or fancoil 4, in replacement of a conventional anti-dust filter, and generally indicated by the reference number 5 in figure 1.

The first construction 2 comprises a box-like body including an electronic hour or time counter 6, a lamp electronic power supply 7, an ionizer power supply 8, a high voltage source 17, or ionizer source, and an electronic control device for controlling the lamp switching on and off.

The front panel of the first construction comprises a display indicator 13 of said hour counter, including, for example LED elements.

The second construction 3 comprises a framework 9, of substantially box-like shape, for holding therein UV lamps 10, the emitter of the air ionizer 11 and an anti-dust filter 12.

The first construction 2 can be coupled to the second construction 3 by coupling pins 14, provided on the first construction 2 and adapted to be engaged in engaging seats 15, including locking spring elements, and formed on the front wall 16 of the second construction 3.

The UV lamps 10 are preferably germicide UVC lamps.

It has been found that the invention fully achieves the intended aim and objects.

In fact, the invention provides an air sterilizing unit, including UV lamp to be applied to ventilation convectors or fancoils, and also including an hour or time counter for controlling the anti-dust filter and the effective lifetime of the germicide tubes, and also including an electronic control device for controlling the germicide lamp switching on and off, and their proper operation, and comprising a negative ion ionizing system.

The air sterilizing unit according to the present invention is installed in replacement of the fancoil existing anti-dust or anti-powder filter.

The modular construction of the subject unit is very advantageous, since it allows to use a first construction, or head construction, having a size which can be fitted to different fancoil structures, for each of which a second construction, filter holder and lamp holder of suitable size is used.

The first construction, or head, can be housed inside the fancoil, even separately from the second filter holder and lamp holder construction.

In practicing the invention, the used materials, as well the contingent size and shapes, can be any, depending on requirements and the status of the art.

## Claims

1. An air sterilizing unit for fancoils and the like, **characterized in that** said air sterilizing unit comprises a construction including air sterilizing means and which can be associated with a further construction including control means for controlling said air sterilizing means, said air sterilizing unit, constituted by said two assembled constructions, being adapted to be arranged in a said fancoil, in replacement of a conventional anti-dust filter.

2. An air sterilizing unit, according to claim 1, **characterized in that** the first construction comprises a box-like body including an electronic hour counter, a lamp electronic power supply, a further electronic device for controlling the switching on and off of the lamps and their proper operation, an ionizer power supply device, and a high voltage source operating as an air ionizer.

3. An air sterilizing unit, according to one or more of the preceding claims, **characterized in that** said electronic hour counter is arranged in the second construction.

4. An air sterilizing unit, according to one or more of the preceding claims, **characterized in that** said lamp electronic power supply is arranged in the second construction.

5. An air sterilizing unit, according to one or more of the preceding claims, **characterized in that** said electronic hour counter is arranged outside of said first construction and said second construction at a different position.

6. An air sterilizing unit, according to one or more of the preceding claims, **characterized in that** said hour counter comprises a display arranged outside of said first construction.

7. An air sterilizing unit, according to one or more of the preceding claims, **characterized in that** said electronic device for controlling said lamp switching on and off and the operation of said lamps comprises a display including LED elements and arranged outside of said first construction and said second construction at a different position.

8. An air sterilizing unit, according to claim 1 or 2, **characterized in that** said first construction comprises a front panel including said hour counter display.

9. An air sterilizing unit, according to claim 3, **characterized in that** said hour counter display comprises LED elements.

10. An air sterilizing unit, according to one or more of the preceding claims, **characterized in that** said first construction comprises an electronic device for controlling the lamp switching on and off and a proper operation of said lamps.

11. An air sterilizing unit, according to one or more of the preceding claims, **characterized in that** said front panel of said first construction comprises a display of said electronic device for controlling the switching on and off of said lamps and the proper operation of said lamps.

12. An air sterilizing unit, according to one or more of the preceding claims, **characterized in that** said display of said electronic device for controlling said lamps switching on and off and the proper operation of said lamps comprises a LED.

13. An air sterilizing unit, according to one or more of the preceding claims, **characterized in that** said electronic device for controlling said lamp switching on and off and the proper operation of said lamps is arranged at a different position in said second construction.

14. An air sterilizing unit, according to one or more of the preceding claims, **characterized in that** said electronic device for controlling said lamp switching on and off and the proper operation of said lamps is arranged outside of said first construction and said second construction, at a different position, depending on the technical-constructional requirements of said fancoils.

15. An air sterilizing unit, according to one or more of the preceding claims, **characterized in that** said second construction comprises a substantially box-like framework, designed for holding UV lamps therein, an air ionizer and an anti-dust powder.

16. An air sterilizing unit, according to one or more of the preceding claims, **characterized in that** said second construction comprises a substantially box-like framework, holding therein UV lamps, the air ionizer emitter and an anti-dust filter.

17. An air sterilizing unit, according to one or more of the preceding claims, **characterized in that** said air ionizer emitter is arranged outside of said second construction.

18. An air sterilizing unit, according to one or more of the preceding claims, **characterized in that** said first construction is coupled to said second construction by coupling pins, provided on said first construction, and engageable in suitably seats, including locking spring elements, formed on a front wall of said second construction.

19. An air sterilizing unit, according to one or more of the preceding claims, **characterized in that** said UV lamps comprise germicide UVC lamps.

20. An air sterilizing unit, according to one or more of the preceding claims, **characterized in that** said hour counter operates as a control element for said filter and the effective lifetime of the germicide lamps.

21. An air sterilizing unit, according to one or more of the preceding claims, **characterized in that** said lamp control electronic device is adapted to control the switching on of said lamps and the proper operation of said lamps.

22. An air sterilizing unit, according to one or more of the preceding claims, **characterized in that** said first construction is arranged inside said fancoil, even separately from the second filter holder-lamp holder construction.
